# EUROPEAN PATENT APPLICATION

(11) **EP 1 524 265 A1**
(43) Date of publication of application: **20.04.2005**
(21) Application number: 03023341.5
(22) Date of filing: 15.10.2003
(51) Int. Cl.: C07D 207/16, C07D 211/60, C07D 223/06, C07D 401/12, A61K 31/401, A61K 31/4025, A61K 31/445, A61K 31/55, A61P 25/00, A61P 1/00, A61P 13/00

(54) **Prolinamide derivatives as sodium and/or calcium channel blockers or selective MAO-B inhibitors**

(71) Applicant: Newron Pharmaceuticals S.p.A., 20091 Bresso (MI) (IT)
(72) Inventor: Caccia, Carla, 20091 Bresso (MI) (IT); La Porta, Elena, 20021 Baranzate di Bollate (MI) (IT); Maestroni, Sara, 20091 Bresso (MI) (IT); Melloni, Piero, 20091 Bresso (MI) (IT); Sabido David, Cibele, 20091 Bresso (MI) (IT); Salvati, Patricia, 20091 Bresso (MI) (IT)
(74) Representative: Minoja, Fabrizio, Dr.

(57) **Abstract**

This invention is related to novel prolinamide derivatives and analogues of general formula **I** 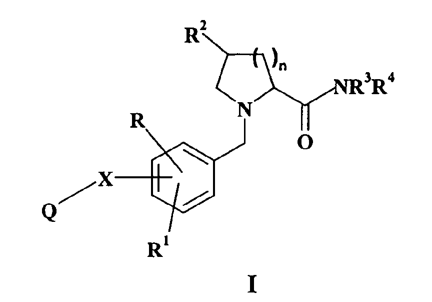 wherein
- Q: is C₃-C₈ alkyl, or C₁-C₈ alkyl substituted by phenoxy or saturated or unsaturated 5 to 7 membered ring optionally containing one or more heteroatoms chosen independently from nitrogen, oxygen or sulphur, the rings being optionally substituted by one or more halogen atoms, trifluoromethyl, C₁-C₆ alkyl, hydroxyl, C₁-C₆ alkoxy;
- X: is an oxygen, sulphur or a NR⁵ group;
- R, R¹: are independently hydrogen, C₁-C₆ alkyl, halogen, trifluoromethyl, hydroxy or C₁-C₆ alkoxy;
- R²,: is hydrogen or hydroxyl;
- R³, R⁴, R⁵: are independently hydrogen or straight or branched C₁-C₃ alkyl;
- n: is an integer from 1 to 3
and a pharmaceutically acceptable salt or prodrug thereof, that are active as sodium and/or calcium channel modulators and/or as selective MAO-B inhibitors and therefore useful in preventing, alleviating and curing a wide range of pathologies, including, but not limited to, neurological, psychiatric, cardiovascular, inflammatory, ophthalmic, urologic, metabolic and gastrointestinal diseases, where the above mechanisms have been described as playing a pathological role.

## Description

This invention is related to novel prolinamide derivatives and analogues of general formula **I** 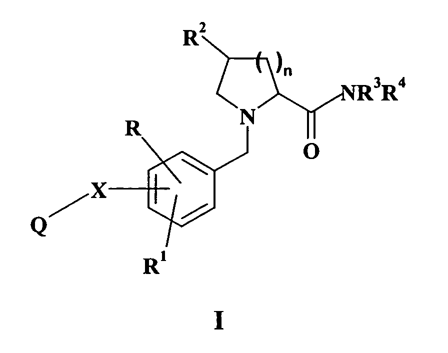 wherein
- Q: is C₃-C₈ alkyl, or C₁-C₈ alkyl substituted by phenoxy or saturated or unsaturated 5 to 7 membered ring optionally containing one or more heteroatoms chosen independently from nitrogen, oxygen or sulphur, the rings being optionally substituted by one or more halogen atoms, trifluoromethyl, C₁-C₆ alkyl, hydroxyl, C₁-C₆ alkoxy;
- X: is an oxygen, sulphur or a NR⁵ group;
- R, R¹: are independently hydrogen, C₁-C₆ alkyl, halogen, trifluoromethyl, hydroxy or C₁-C₆ alkoxy;
- R²,: is hydrogen or hydroxyl;
- R³, R⁴, R⁵: are independently hydrogen or straight or branched C₁-C₃ alkyl;
- n: is an integer from 1 to 3
and a pharmaceutically acceptable salt or prodrug thereof, that are active as sodium and/or calcium channel modulators and/or as selective MAO-B inhibitors and therefore useful in preventing, alleviating and curing a wide range of pathologies, including, but not limited to, neurological, psychiatric, cardiovascular, inflammatory, ophthalmic, urologic, metabolic and gastrointestinal diseases, where the above mechanisms have been described as playing a pathological role.

### BACKGROUND OF THE INVENTION

### Chemical background

WO 90/14334, WO 94/22808, WO 94/22809, WO 98/43964 and WO 97/05102 disclose aminoalkyl-carboxamide derivatives and WO 97/05111 discloses N-(4-substituted-benzyl)-2-aminolactam derivatives. The compounds are disclosed to be useful as antiepileptics, and as neuroprotective agents in treating and preventing neurodegenerative diseases such as Parkinson's disease, stroke, hypoxia, ischemia, CNS trauma, hypoglycemia or surgery, Alzheimer's disease, amyotrophic lateral sclerosis, Down's syndrome, Huntington's disease, dementia caused by acquired immunodeficiency syndrome (AIDS), multinfarctual dementia and infections or inflammations in the brain; they can also be used as antidepressants, hypnotics, and anti spastic agents and in treating ocular damage and retinopathy. WO 99/26614 relates to substituted aminoalkyl-carboxamide derivatives which, by blocking the sodium (Na⁺) channels, may be useful as local anaesthetics or for treating, preventing or ameliorating neuronal loss following global and focal ischemia, neurodegenerative conditions, pain, manic depression or arrhythmias. WO 99/35123 and WO 99/35125 relate to substituted aminoalkyl-carboxamide derivatives as analgesic agents (pain).
WO 00/33788 relate to substituted nitrogen heterocyclic compounds for treating or controlling the symptoms of memory loss, Alzheimer's disease, senile dementia or similar conditions. WO 02/44126 relates to bi-substituted carbocyclic cyclophilins and their use in a variety of medical disorders, among them neurological disorders.

### Biological background

It is well known that sodium channels play an important role in the neuronal network by transmitting electrical impulses rapidly throughout cells and cell networks, thereby coordinating higher processes ranging from locomotion to cognition. These channels are large transmembrane proteins, which are able to switch between different states to enable selective permeability for sodium ions. For this process an action potential is needed to depolarize the membrane, and hence these channels are voltage-gated. In the past few years a much better understanding of sodium channels and drugs interacting with them has been developed.

It has become clear that a number of drugs having an unknown mechanism of action actually act by modulating sodium channel conductance, including local anesthetics, class I antiarrhythmics and anticonvulsants. Neuronal sodium channel blockers have found application with their use in the treatment of epilepsy (phenytoin and carbamazepine), bipolar disorder (lamotrigine), preventing neurodegeneration, and in reducing neuropathic pain. Various anti-epileptic drugs that stabilize neuronal excitability are effective in neuropathic pain (gabapentin).

In addition, an increase in sodium channel expression or activity has also been observed in several models of inflammatory pain, suggesting a role of sodium channels in inflammatory pain.

Calcium channels are membrane-spanning, multi-subunit proteins that allow controlled entry of calcium ions into cells from the extracellular fluid. Commonly, calcium channels are voltage dependent and are referred to as voltage sensitive calcium channels (VSCC). VSCCs are found throughout the mammalian nervous system, where they regulate such varied activities as cellular excitability, transmitter release, intracellular metabolism, neurosecretory activity and gene expression. All "excitable" cells in animals, such as neurons of the central nervous system (CNS), peripheral nerve cells, and muscle cells, including those of skeletal muscles, cardiac muscles and venous and arterial smooth muscles, have voltage dependent calcium channels. Calcium channels have a central role in regulating intracellular calcium ions levels that are important for cell viability and function. Intracellular calcium ion concentrations are implicated in a number of vital processes in animals, such as neurotransmitter release, muscle contraction, pacemaker activity, and secretion of hormones. It is believed that calcium channels are relevant in certain disease states. A number of compounds useful in treating various cardiovascular diseases in mammals, including humans, are thought to exert their beneficial effects by modulating functions of voltage dependant calcium channels present in cardiac and/or vascular smooth muscle. Compounds with activity against calcium channels have also been implicated for the treatment of pain. In particular N-type calcium channels (Cav2.2), responsible for the regulation of neurotransmitters, are thought to play a significant role in nociceptive transmission, both due to their tissue distribution as well as from the results of several pharmacological studies. This hypothesis has been validated in the clinic by Zinocotide, a peptide derived from the venom of the marine snail, Conus Magus. A limitation in the therapeutic use of this peptide is that it has to be administered intrathecally in humans (Bowersox S. S. and Luther R. Toxicon, 1998, 36, 11, 1651-1658).

Monoamine oxidase (MAO) is an integral protein of the outer mitochondrial membrane and plays a major role in the in vivo inactivation of biogenic and diet-derived amines in both the CNS and in peripheral neurons and tissues. Two MAO enzymes are distinguished on the basis of their substrate preferences and sensitivity to inhibition by the MAO inhibitor, clorgyline:
- MAO type A (MAO-A) which in the human CNS is responsible for the deamination of serotonin and noradrenaline. The highest MAO-A concentrations are in the catecholaminergic neurons of the locus ceruleus;
- MAO-B which is responsible mainly for the catabolism of dopamine. In contrast to the rat brain, MAO-B is the major form in the human and guinea pig CNS. The highest MAO-B concentrations are in the serotoninergic neurons of the raphe nucleus and posterior hypothalamus. The nigral MAO-B is located primarily in glial cells.

MAO-B (but not MAO-A) activity in CNS increases with age in both humans and animals, perhaps as a result of the glial cell proliferation associated with neuronal loss. Increased MAO-B levels in Alzheimer's plaques have also been reported. Increased blood platelet MAO-B activity has been reported in both Alzheimer's (AD) and Parkinson's Disease (PD). MAO-B activity was reduced by 40% in the brain of smokers: tobacco use is associated both with psychiatric disease and with a reduced risk for PD.

Most currently investigated MAO-B inhibitors are irreversible. The inhibition is very persistent (weeks), as its effects can only be overcome by de novo synthesis of the enzyme. Interest in the MAO-B inhibition was initially stimulated by the desire to elevate the reduced striatal DA concentrations characteristic of PD. As increased DA concentration in the synaptic cleft would be expected as the primary effect of treatment with a selective MAO-B inhibitor. In PD, the need to supply the DA precursor L-Dopa, the golden standard PD therapy, should thus be reduced. This is desirable, as L-Dopa, despite the excellent initial improvement achieved, is associated in the longer term with increasingly severe side effects, including motors fluctuations, dyskinesia and dystonia.

In addition to loss of cholinergic neurons, there is a decrease in the levels of the DA, noradrenaline and serotonin in the brains of AD patients. MAO-B activity is significantly increased in the platelets and selected brain regions of AD patients. MAO-B inhibitors may act by both reducing the formation of oxygen radicals and preventing the breakdown of monoamines and thus elevating their level in the brain of AD patients.

Promising results using MAO-B inhibitors were reported also for the treatment of narcolepsy, Tourette's syndrome/attention deficit disorders, negative symptoms of schizophrenia, drug addiction and obesity.

All together these findings indicate that compounds with sodium and/or calcium channel blockade and/or MAO-B inhibition have a high therapeutic potential in preventing, alleviating and curing a wide range of pathologies, including neurological, psychiatric, cardiovascular, urologic, metabolic and gastrointestinal diseases, where the above mechanisms have been described as playing a pathological role.

There are many papers and patents which describe sodium channel and/or calcium channel modulators or antagonists and/or MAO-B inhibitors for the treatment or modulation of a plethora of disorders, such as their use as local anesthetics, antiarrhythmics, antiemetics, antimanic depressants, agents for the treatment of unipolar depression, cardiovascular diseases, urinary incontinence, diarrhea, inflammation, epilepsy, neurodegenerative conditions, nerve cell death, anticonvulsants, neuropathic pain, migraine, acute hyperalgesia and inflammation, renal disease, allergy, asthma, bronchospasm, dysmenorrhea, esophageal spasm, glaucoma, urinary tract disorders, gastrointestinal motility disorders, premature labour, obesity. A largely incomplete list is shown below.

An extensive and thorough prior art overview is reported in WO 03/057219 (and references therein); a further selection of prior art is reported in the following references: Alzheimer, C. Adv. Exp. Med. Biol. 2002, 513, 161-181; Vanegas, H.; Schaible, H. Pain 2000, 85, 9-18; U.S. Patent 5,051,403; U.S. Patent 5,587,454; U.S. Patent 5,863,952; U.S. Patent 6,011,035; U.S. Patent 6,117,841; U.S. Patent 6,362,174; U.S. Patent 6,380,198; U.S. Patent 6,420,383; U.S. Patent 6,458,781; U.S. Patent 6,472,530; U.S. Patent 6,518,288; U.S. Patent 6,521,647; WO 97/10210; WO 03/018561; WO 01/12176; WO 96/37199; U.S. patent 6, 210,706.

### DESCRIPTION OF THE INVENTION

The present invention provides novel compounds of formula **I** 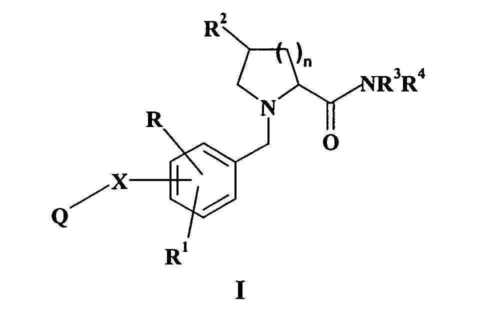 wherein
- Q: is C₃-C₈ alkyl, or C₁-C₈ alkyl substituted by phenoxy or saturated or unsaturated 5 to 7 membered ring optionally containing one or more heteroatoms chosen independently from nitrogen, oxygen or sulphur, the rings being optionally substituted by one or more halogen atoms, trifluoromethyl, C₁-C₆ alkyl, hydroxyl, C₁-C₆ alkoxy;
- X: is an oxygen, sulphur or a NR⁵ group;
- R, R¹: are independently hydrogen, C₁-C₆ alkyl, halogen, trifluoromethyl, hydroxy or C₁-C₆ alkoxy;
- R²,: is hydrogen or hydroxyl;
- R³, R⁴, R⁵: are independently hydrogen or straight or branched C₁-C₃ alkyl;
- n: is an integer from 1 to 3
and a pharmaceutically acceptable salt thereof.

The pharmaceutically acceptable salts of the compounds of formula **I** include acid addition salts with inorganic, e.g. hydrochloric, hydrobromic, sulphuric, and phosphoric acids or organic, e.g. acetic, propionic, benzoic, cinnamic, mandelic, salicylic, glycolic, lactic, oxalic, malic, maleic, malonic, fumaric, tartaric, citric, and the like.

Examples of specific compounds of the invention are:
(S)-1-[2-(3-Fluoro-benzyloxy)-benzyl]-pyrrolidine-2-carboxylic acid amide;
(S)-1-[2-(3-Chloro-benzyloxy)-benzyl]-pyrrolidine-2-carboxylic acid amide;
(S)-1-[3-(3-Fluoro-benzyloxy)-benzyl]-pyrrolidine-2-carboxylic acid amide;
(S)-1-[3-(3-Chloro-benzyloxy)-benzyl]-pyrrolidine-2-carboxylic acid amide;
(S)-1-[4-(3-Fluoro-benzyloxy)-benzyl]-pyrrolidine-2-carboxylic acid amide;
(S)-1-[4-(3-Chloro-benzyloxy)-benzyl]-pyrrolidine-2-carboxylic acid amide;
(2S)-1-[4-(3-Fluoro-benzyloxy)-benzyl]-4-hydroxy-pyrrolidine-2-carboxylic acid amide;
(2S)-1-[4-(3-Chloro-benzyloxy)-benzyl]-4-hydroxy-pyrrolidine-2-carboxylic acid amide;
(S)-1-[4-(3-Fluoro-benzyloxy)-benzyl]-piperidine-2-carboxylic acid amide;
(S)-1-[4-(3-Chloro-benzyloxy)-benzyl]-piperidine-2-carboxylic acid amide;
(S)-1-[4-(3-Fluoro-benzyloxy)-benzyl]-azepine-2-carboxylic acid amide;
(S)-1-[4-(3-Chloro-benzyloxy)-benzyl]-azepine-2-carboxylic acid amide;
(S)-1-[4-(3-Chloro-benzyloxy)-benzyl]-pyrrolidine-2-carboxylic acid N-methyl amide;
(S)-1-[4-(3-triFluoromethyl-benzyloxy)-benzyl]-pyrrolidine-2-carboxylic acid amide;
(S)-1-[4-(3-triChloromethyl-benzyloxy)-benzyl]-pyrrolidine-2-carboxylic acid amide;
(S)-1-[4-(3,4-di-Chloro-benzyloxy)-benzyl]-pyrrolidine-2-carboxylic acid amide;
(S)-1-[4-(3-Chloro-benzyloxy)-2-chlorobenzyl]-pyrrolidine-2-carboxylic acid amide;
(S)-1-[4-(2-Fluoro-benzyloxy)-3-chlorobenzyl]-pyrrolidine-2-carboxylic acid amide;
(S)-1-[4-(2-Fluoro-benzyloxy)-2-methoxybenzyl]-pyrrolidine-2-carboxylic acid amide;
(S)-1-[4-(2-Fluoro-benzyloxy)-3-methoxybenzyl]-pyrrolidine-2-carboxylic acid amide;
(S)-1-[4-(2-Fluoro-benzyloxy)-3-bromo-5-methoxybenzyl]-pyrrolidine-2-carboxylic acid amide;
(S)-1-[4-(2-Fluoro-benzyloxy)-3-fluorobenzyl]-pyrrolidine-2-carboxylic acid amide;
(S)-1-[4-(3-triFluoromethyl-benzyloxy)-2-chlorobenzyl]-pyrrolidine-2-carboxylic acid amide;
(S)-1-[4-(Pyridin-2-ylmethoxy)-benzyl]-pyrrolidine-2-carboxylic acid amide;
(S)-1-[4-(Pyridin-3-ylmethoxy)-benzyl]-pyrrolidine-2-carboxylic acid amide;
(S)-1-[4-(Pyridin-4-ylmethoxy)-benzyl]-pyrrolidine-2-carboxylic acid amide;
and the corresponding (R) enantiomers and the pharmaceutically acceptable salts thereof.

The compounds of the invention and the salts thereof can be obtained by a process comprising:
a) reaction of compounds of formula **II** 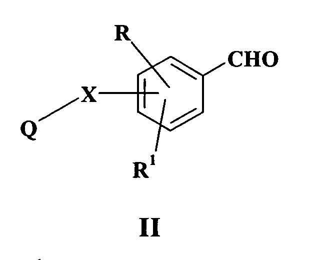 wherein Q, X, R and R¹ are as defined above
   with compounds of formula **III,** in the presence of a reducing agent 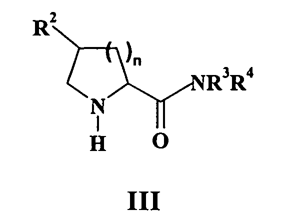 wherein R², R³, R⁴ and n are as defined previously thus obtaining a compound of formula **I**; or
b) reaction of compounds of formula **IV** 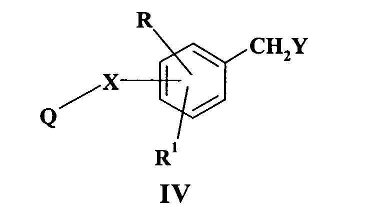
wherein Q, X, R and R¹ are as defined above and Y is a halogen atom or a O-EWG group, where the EWG means an electron withdrawing group, like e.g. mesyl, tosyl or trifluoroacetyl groups, able to transform the oxygen which they are linked to, in a good leaving group
with compounds of formula **III** and, if desired, converting a compound of the invention into another compound of the invention and/or, if desired, converting a compound of the invention into a pharmaceutically acceptable salt and/or, if desired, converting a salt into a free compound.

Compounds **II-IV** are commercially available compounds or are prepared from commercially available compounds using well-known methods.

The reaction of compounds of formula **II** with compounds of formula **III** to give the compounds of formula **I** is a reductive amination reaction which can be carried out according to well known methods. According to a preferred embodiment of the invention, it may be performed under nitrogen atmosphere, in a suitable organic solvent, such as an alcohol, e.g. a lower alkanol, in particular methanol, or in acetonitrile, at a temperature ranging from about 0°C to about 40°C, in the presence of a reducing agent, the most appropriate being sodium cyanoborohydride. Occasionally molecular sieves can be added to the reaction mixture for facilitating the reaction.

In a compound of formula **IV** the halogen is preferably bromine or iodine. The alkylation reaction of a compound of formula **IV** with a compound of formula **III** can be carried out in a suitable organic solvent, such as an alcohol, e.g. methanol, ethanol or isopropanol, in particular in ethanol, at a temperature ranging from about 0°C to about 50°C.

When in the compounds of the present invention and in the intermediate-products thereof, groups are present, which need to be protected before submitting them to the above illustrated reactions, they may be protected before being reacted and then deprotected according to well-known methods.

### PHARMACOLOGY

The compounds of the invention display affinities for the calcium and/or sodium channel binding sites as demonstrated using selective radioligands in *in vitro* binding studies.

The compounds according to the invention are blockers of the voltage dependent sodium channels and/or calcium channels. These compounds therefore displace 3H-batrachotoxin (BTX) with a high affinity from the binding site on the sodium channel, with IC₅₀ in the low µM or sub µM range. Similarly the compounds displace 3H-nitrendipine from the binding site in the calcium channel, with IC₅₀ in the low µM or most usually in the sub µM range as well as inhibiting calcium influx induced through calcium channels via cellular depolarisation.

Such substances exhibit "use-dependency" when the sodium channels are blocked i.e. maximum blockage of the sodium channels is only achieved after repeated stimulation of the sodium channel. Consequently, the substances preferably bind to sodium channels which are multiply activated. As a result the substances are capable of activity preferentially in those regions of the body which are pathologically over-stimulated, as illustrated by patch-clamp experiments (W.A. Catteral, Trends Pharmacol. Sci., 8, 57-65; 1987) which show that the compounds according to the invention block the electrically stimulated sodium channel in a "use-dependent" manner.

As a consequence of these mechanisms the compounds of the invention are active *in vivo* when orally administered in the range of 0.1 to 100 mg/kg in a wide range of animal models and in particular in the MES test of electrically-induced convulsions, in the formalin model of persistent pain and in the carragenan model of inflammation.

The compounds of this invention beyond being potent calcium and/or sodium channels blockers are also able to selectively and reversibly inhibit MAO-B both *in vitro* and *in vivo.*

The compounds of the invention that are potent inhibitors of MAO-B (IC₅₀ in the submicromolar range) have generally no relevant effect on MAO-A. In enzymatic studies using rat brain membranes and selective radiolabelled substrates, the compounds are generally over 1000 times more active as MAO-B inhibitors than MAO-A inhibitors. The MAO-B inhibition by the compounds is not time-dependent and the enzyme activity fully recovers after washings as it is typical for reversible inhibitors.

After oral administration in mice, the compounds behave as potent and reversible, short-acting MAO-B inhibitors with full recovery of activity 24 h after treatment.

In view of the above described mechanisms of action, the compounds of the present invention are useful in the treatment or prevention of neuropathic pain. Neuropathic pain syndromes include, and are not limited to: diabetic neuropathy; sciatica; non-specific lower back pain; multiple sclerosis pain; fibromyalgia; HIV-related neuropathy; neuralgia, such as post-herpetic neuralgia and trigeminal neuralgia; and pain resulting from physical trauma, amputation, cancer, toxins or chronic inflammatory conditions.

Compounds of the invention are also useful for the treatment of chronic pain. Chronic pain includes, and is not limited to, chronic pain caused by inflammation or an inflammatory-related condition, ostheoarthritis, rheumatoid arthritis or as sequela to disease, acute injury or trauma and includes upper back pain or lower back pain (resulting from systematic, regional or primary spine disease (such as radiculopathy), bone pain (due to osteoarthritis, osteoporosis, bone metastasis or unknown reasons), pelvic pain, spinal cord injury-associated pain, cardiac chest pain, non-cardiac chest pain, central post-stroke pain, myofascial pain, cancer pain, AIDS pain, sickle cell pain, geriatric pain or pain caused by headache, temporomandibular joint syndrome, gout, fibrosis or thoracic outlet syndromes.

Compounds of the invention are also useful in the treatment of acute pain (caused by acute injury, illness, sports-medicine injuries, carpal tunnel syndrome, burns, musculoskeletal sprains and strains, musculotendinous strain, cervicobrachial pain syndromes, dyspepsis, gastric ulcer, duodenal ulcer, dysmenorrhea, endometriosis or surgery (such as open heart or bypass surgery), post operative pain, kidney stone pain, gallbladder pain, gallstone pain, obstetric pain or dental pain.

Compounds of the invention are also useful in the treatment of migraine, such as tension type headache, transformed migraine or evolutive headache, cluster headache, as well as secondary headache disorders, such as the ones derived from infections, metabolic disorders or other systemic illnesses and other acute headaches, paroxysmal hemicrania and the like, resulting from a worsening of the above mentioned primary and secondary headaches.

Compounds of the invention are also useful for the treatment of neurological conditions and cognitive disorders. Neurological conditions include, and are not limited to, conditions such as epilepsy (including simple partial seizures, complex partial seizures, secondary generalised seizures, further including absence seizures, myoclonic seizures, clonic seizures, tonic seizures, tonic clonic seizures and atonic seizures), degenerative dementia (including senile dementia, Alzheimer's disease, Pick's disease, Huntington's chorea, Parkinson's disease and Creutzfeldt-Jakob disease), and vascular dementia (including multi-infarct dementia, stroke and cerebral ischemia), as well as dementia associated with intracranial space occupying lesions, trauma, infections and related conditions (including HIV infection), metabolism, toxins, anoxia and vitamin deficiency; and mild cognitive impairment associated with aging, particularly age associated memory impairment, movement disorders (postencephalitic parkinsonism, progressive supranuclear palsy, corticobasal degeneration, restless leg syndrome), narcolepsy, deficit and hyperactivity disorders (ADHD), Tourette's Syndrome, amyotrophic lateral sclerosis, Down's syndrome.

Compounds of the invention are also useful for the treatment of psychiatric disorders. Psychiatric disorders include, and are not limited to, manic depression also known as bipolar disorder (such as bipolar disorder type I, bipolar disorder type II), cyclothymic disorder, rapid cycling, ultradian cycling, bipolar depression, acute mania, mania, mixed mania, hypomania or unipolar depression, schizophrenia, schizophreniform disorders, schizoaffective disorders, delusional disorders, brief psychotic disorders, shared psychotic disorders, psychotic disorder due to a general medical condition, substance-induced psychotic disorders or a psychotic disorder not otherwise specified, anxiety disorders and moreover in smoke and drug addiction.

Compounds of the invention are also useful in the treatment of peripheral diseases such as tinnitus, muscle spasm, muscular sclerosis, and other disorders, including, and not limited to cardiovascular diseases (such as cardiac arrhythmia, cardiac infarction or angina pectoris, hypertension, hypoxia, cardiac ischemia) endocrine disorders (such as acromegaly or diabetes insipidus), diseases in which the pathophysiology of the disorder involves excessive or hypersecretory or otherwise inappropriate cellular secretion of an endogeneous substance (such as catecholamine, a hormone or a growth factor).

Compounds of the invention are also useful in the treatment of liver disease, such as inflammatory liver disease, for example chronic viral hepatitis B, chronic viral hepatitis C, alcoholic liver injury, primary biliary cirrhosis, autoimmune hepatitis, non-alcoholic steatohepatitis and liver transplant rejection.

Compounds of the invention inhibit inflammatory processes affecting all body systems. Therefore are useful in the treatment of inflammatory processes of the muscular-skeletal system of which the following is a list of exemples but it is not comprehensive of all target disorders: arthritic conditions such as alkylosing spondylitis, cervical arthritis, fibromyalgia, gut, juvenile rheumatoid arthritis, lumbosacral arthritis, osteoarthritis, osteoporosis, psoriatic arthritis, rheumatic disease; disorders affecting skin and releted tissues: eczema, psoriasis, dermatitis and inflammatory conditions such as sunburn; disorders of the respiratory system: asthma, allergic rhinitis and respiratory distress syndrome, lung disorders in which inflammation is involved such as asthma and bronchitis; chronic obstructive pulmonary disease; disorders of the immune and endocrinological systems: periarteritis nodosa, thyroiditis, aplastic anaemia,, sclerodoma, myasthenia gravis, multiple sclerosis, sarcoidosis, nephritic syndrome, Bechet's syndrome, polymyositis, gingivitis.

Compounds of the invention are also useful in the treatment of gastrointestinal (GI) tract disorders such as inflammatory bowel disorders including but not limited to ulcerative colitis, Crohn's disease, ileitis, proctitis, celiac disease, enteropathies, microscopic or collagenous colitis, eosinophilic gastroenteritis, or pouchitis resulting after proctocolectomy and post ileonatal anastomosis, and irritable bowel syndrome including any disorders associated with abdominal pain and/or abdominal discomfort such as pylorospasm, nervous indigestion, spastic colon, spastic colitis, spastic bowel, intestinal neurosis, functional colitis, mucous colitis, laxative colitis and functional dyspepsia; but also for treatment of atrophic gastritis, gastritis varialoforme, ulcerative colitis, peptic ulceration, pyresis, and other damage to the GI tract, for example, by *Helicobacter pylori,* gastroesophageal reflux disease, gastroparesis, such as diabetic gastroparesis; and other functional bowel disorders, such as non-ulcerative dyspepsia (NUD); emesis, diarrhea, and visceral inflammation.

Compounds of the invention are also useful in the treatment of disorders of the genito-urinary tract such as overactive bladder, prostatitis (chronic bacterial and chronic non-bacterial prostatitis), prostadynia, interstitial cystitis, urinary incontinence and benign prostatic hyperplasia, annexitics, pelvic inflammation, bartolinities and vaginitis.

Compounds of the invention are also useful in the treatment of ophthalmic diseases such as retinitis, retinopathies, uveitis and acute injury to the eye tissue, macular degeneration or glaucoma, conjunctivitis.

Compounds of the invention are also useful in the treatment of obesity.

Compounds of the invention are also useful for the treatment of all other conditions mediated by the inhibition of voltage gated sodium channels and/or voltage gated calcium channels and/or MAO-B.

It will be appreciated that the compounds of the invention may advantageously be used in conjunction with one or more other therapeutic agents. Examples of suitable agents for adjunctive therapy include L-Dopa or a dopamine agonists, a 5HT_{1B/1D} agonist, such as a triptan (e.g. sumatriptan or naratriptan); an adenosine A1 agonist; an EP ligand; an NMDA modulator, such as a glycine antagonist; a substance P antagonist (e.g. an NK1 antagonist); a cannabinoid; acetaminophen or phenacetin; a 5-lipoxygenase inhibitor; a leukotriene receptor antagonist; a DMARD (e.g. methotrexate); gabapentin and related compounds; a tricyclic antidepressant (e.g. amitryptiline); a neurone stabilising antiepileptic drug; a monoaminergic uptake inhibitor (e.g. venlafaxine); a matrix metalloproteinase inhibitor; a nitric oxide synthase (NOS) inhibitor, such as an iNOS or an nNOS inhibitor; an inhibitor of the release, or action, of tumor necrosis factor alpha; an antibody therapy, such as monoclonal antibody therapy; an antiviral agent, such as a nucleoside inhibitor (e.g. (lamivudine) or an immune system modulator (e.g. interferon); an analgesic, such as a a cyclooxygenase-2 inhibitor; a local anaesthetic; a stimulant, including caffeine; an H2-antagonist (e.g. ranitidine); a proton pump inhibitor (e.g. omeprazole); an antacid (e.g. aluminium or magnesium hydroxide; an antiflatulent (e.g. semethicone); a decongestant (e.g. phenylephrine, phenylpropanolamine, pseudoephedrine, oxymetazoline, epinephrine, naphazoline, xylometazoline, propylhexedrine, or levo-desoxyephedrine,naphazoline, xylometazoline, propylhexedrine, or levodesoxyephedrine); antitussive (e.g. codeine, hydrocodone, carmiphen, carbetapentane, or dextramethorphan); a diuretic; or a sedating or non-sedating antihistamine. It is to be understood that the present invention covers the use of a compound of formula (I) or a pharmaceutically acceptable salt thereof in combination with one or more therapeutic agents.

The compounds of the present invention are useful in human and veterinary medicine. It is to be understood that reference to treatment includes both treatments of established symptoms and prophylactic treatment, unless explicitly stated otherwise.

The prolinamide derivatives and analogues of formula I as above defined can be administered as the "active ingredient" of a pharmaceutically acceptable composition which can be prepared by conventional procedures, for instance by mixing the active ingredient with pharmaceutically acceptable, therapeutically inert organic and/or inorganic carrier materials.

The composition comprising the above defined prolinamide derivatives and analogues can be administered in a variety forms, e.g. orally, in the form of tablets, troches, capsules, sugar or film coated tablets, liquid solutions, emulsions or suspensions; rectally, in the form of suppositories; parenterally, e.g. by intramuscular or intravenous injection or infusion; and transdermally.

Suitable pharmaceutically acceptable, therapeutically inert organic and/or inorganic carrier materials useful in the preparation of such composition include, for example, water, gelatin, gum arabic, lactose, starch, cellulose, magnesium stearate, talc, vegetable oils, polyalkyleneglycols and the like. The composition comprising the prolinamide derivatives and analogues of formula I as above defined can be sterilized and may contain further well known components, such as, for example, preservatives, stabilizers, wetting or emulsifying agents, e.g. paraffin oil, mannide monooleate, salts to adjust osmotic pressure, buffers and the like.

For example, the solid oral forms may contain, together with the active ingredient, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disgregating agents, e.g. a starch, alginic acid, alginates or sodium starch glycolate; effervescing mixtures; dyestuffs; sweeteners; wetting agents such as lecithin, polysorbates, laurylsulphates; and, in general, nontoxic and pharmacologically inactive substances used in pharmaceutical formulations. Said pharmaceutical preparations may be manufactured in known manner, for example, by means of mixing, granulating, tabletting, sugar-coating, or film-coating processes.

The oral formulations comprise sustained release formulations that can be prepared in conventional manner, for instance by applying an enteric coating to tablets and granules.

The liquid dispersion for oral administration may be e.g. syrups, emulsions and suspensions.

The syrups may contain as carrier, for example, saccharose or saccharose with glycerine and/or mannitol and/or sorbitol.

Suspensions and emulsions may contain as a carrier, for example, a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusion may contain as carrier, for example, sterile water or preferably they may be in the form of sterile, aqueous, isotonic saline solutions.

The suppositories may contain, together with the active ingredient, a pharmaceutically acceptable carrier, e.g. cocoa butter, polyethylene glycol, a polyoxyethylene sorbitan fatty acid ester surfactants or lecithin.

The composition comprising the prolinamide derivatives and analogues of formula **I** as above defined is generally in the form of a dose unit containing, for example, 0.1 mg to 100 mg of active ingredient per unit dosage form.

Suitable treatment is given 1, 2 or 3 times daily, depending upon clearance rate. Accordingly, the desired dose may be presented in a single dose or as divided doses administered at appropriate intervals, for example two to four or more sub-doses per day.

The pharmaceutical compositions comprising the prolinamide derivatives and analogues of formula I as above defined will contain, per dosage unit, e.g., capsule, tablet, powder injection, teaspoonful, suppository and the like from about 0.1 to about 500 mg of the active ingredient most preferably from 1 to 10 mg.

Optimal therapeutically effective doses to be administered may be readily determined by those skilled in the art and will vary, basically, with the strength of the preparation, with the mode of administration and with the advancement of the condition or disorder treated. In addition, factors associated with the particular subject being treated, including subject age, weight, diet and time of administration, will result in the need to adjust the dose to an appropriate therapeutically effective level.

The invention is illustrated in more detail in the following examples.

### Example 1

### (S)-(+)-1-[4-(3-Chloro-benzyloxy)-benzyl]-pyrrolidine-2-carboxylic acid amide

To a mixture of 11.4 g (0.1 moles) of prolinamide and 5 g (0.080 moles) of sodium cyanoborohydride in 250 ml of methanol, 22.2 g (0.09 moles) of 4-(3-chloro-benzyloxy)-benzaldehyde are added under stirring. After 3 h at room temperature, the reaction has gone to completeness. The solvent is evaporated to dryness under vacuum and the residue is taken up with water and ethyl acetate, the aqueous solution is extracted three more times, the organic one is washed with brine, dried with anhydrous sodium sulphate and evaporated to dryness.

The residue is passed on column chromatography (flash) using CHCl₃:CH₃OH:NH₃ 95:5:0.5 as an eluant. After evaporation of the solvent 18.2 g of the title compound were obtained as a white solid: m.p. 118-120°C [α]_{D}²⁵ -30.3 (c=1.0, AcOH)

¹H-NMR (500 MHz; DMSO-d6) δ (ppm): 1.58-1.75 (m, 3 H); 1.98-2.08 (m, 1H); 2.15-2.23 (m, 1H); 2.78-2.86 (m, 1H); 2.87-2.94 (m, 1H); 3.27-3.33 (d, 1H, J = 2.5 Hz); 3.73-3.79 (d, 1H, J = 2.5 Hz); 5.08-5.13 (s, 2H); 6.92-6.97 (d, 2H, J = 1.25 Hz); 7.03-7.09 (s, 1H); 7.18-7.24 (s, 1H); 7.25-7.30 (d, 1H, J = 1.25 Hz); 7.35-7.45 (m, 3H); 7.48-7.54 (s, 1H).

Similarly, the following compounds were prepared:
(S)-1-[2-(3-Fluoro-benzyloxy)-benzyl]-pyrrolidine-2-carboxylic acid amide;
(S)-1-[2-(3-Chloro-benzyloxy)-benzyl]-pyrrolidine-2-carboxylic acid amide;
(S)-1-[3-(3-Fluoro-benzyloxy)-benzyl]-pyrrolidine-2-carboxylic acid amide;
(S)-1-[3-(3-Chloro-benzyloxy)-benzyl]-pyrrolidine-2-carboxylic acid amide;
(S)-1-[4-(3-Fluoro-benzyloxy)-benzyl]-pyrrolidine-2-carboxylic acid amide;
(S)-1-[4-(3-Chloro-benzyloxy)-benzyl]-pyrrolidine-2-carboxylic acid amide;
(2S)-1-[4-(3-Fluoro-benzyloxy)-benzyl]-4-hydroxy-pyrrolidine-2-carboxylic acid amide;
(2S)-1-[4-(3-Chloro-benzyloxy)-benzyl]-4-hydroxy-pyrrolidine-2-carboxylic acid amide;
(S)-1-[4-(3-Fluoro-benzyloxy)-benzyl]-piperidine-2-carboxylic acid amide;
(S)-1-[4-(3-Chloro-benzyloxy)-benzyl]-piperidine-2-carboxylic acid amide;
(S)-1-[4-(3-Fluoro-benzyloxy)-benzyl]-azepine-2-carboxylic acid amide;
(S)-1-[4-(3-Chloro-benzyloxy)-benzyl]-azepine-2-carboxylic acid amide;
(S)-1-[4-(3-Chloro-benzyloxy)-benzyl]-pyrrolidine-2-carboxylic acid N-methyl amide;
(S)-1-[4-(3-Trifluoromethyl-benzyloxy)-benzyl]-pyrrolidine-2-carboxylic acid amide;
(S)-1-[4-(3-Trichloromethyl-benzyloxy)-benzyl]-pyrrolidine-2-carboxylic acid amide;
(S)-1-[4-(3,4-di-Chloro-benzyloxy)-benzyl]-pyrrolidine-2-carboxylic acid amide;
(S)-1-[4-(3-Chloro-benzyloxy)-2-chlorobenzyl]-pyrrolidine-2-carboxylic acid amide;
(S)-1-[4-(2-Fluoro-benzyloxy)-3-chlorobenzyl]-pyrrolidine-2-carboxylic acid amide;
(S)-1-[4-(2-Fluoro-benzyloxy)-2-methoxybenzyl]-pyrrolidine-2-carboxylic acid amide;
(S)-1-[4-(2-Fluoro-benzyloxy)-3-methoxybenzyl]-pyrrolidine-2-carboxylic acid amide;
(S)-1-[4-(2-Fluoro-benzyloxy)-3-bromo-5-methoxybenzyl]-pyrrolidine-2-carboxylic acid amide;
(S)-1-[4-(2-Fluoro-benzyloxy)-3-fluorobenzyl]-pyrrolidine-2-carboxylic acid amide;
(S)-1-[4-(3-Trifluoromethyl-benzyloxy)-2-chlorobenzyl]-pyrrolidine-2-carboxylic acid amide;
(S)-1-[4-(Pyridin-2-ylmethoxy)-benzyl]-pyrrolidine-2-carboxylic acid amide;
(S)-1-[4-(Pyridin-3-ylmethoxy)-benzyl]-pyrrolidine-2-carboxylic acid amide;
(S)-1-[4-(Pyridin-4-ylmethoxy)-benzyl]-pyrrolidine-2-carboxylic acid amide;
and the corresponding (R) enantiomers.

### Example 2

### In vitro MAO-A and MAO-B enzyme activities assay

***Membrane preparations (crude mitochondrial fraction):*** male Wistar rats (Harlan, Italy - 175-200 g) were sacrificed under light anaesthesia and brains were rapidly removed and homogenized in 8 vol. of ice-cold 0.32 M sucrose buffer containing 0.1 M EDTA, pH 7.4. The crude homogenate was centrifuged at 2220 rpm for 10 min and the supernatant recovered. The pellet was homogenized and centrifuged again and the two supernatants were pooled and centrifuged at 9250 rpm for 10 min, +4°C. The pellet was resuspended in fresh buffer and centrifuged at 11250 rpm for 10 min, +4°C. The resulting pellet was stored at -80°C until use.

***In vitro enzyme activities assay:*** the enzyme activities were assessed with a radioenzymatic assay using the selective substrates ¹⁴C-serotonin (5-HT) and ¹⁴C-phenylethylamine (PEA) for MAO-A and MAO-B, respectively.

The mitochondrial pellet (500 µg protein) was resuspended in 0.1M phosphate buffer pH 7.4 and 500 µl was added to 50 µl of the test compound or buffer for 30 min at 37°C (preincubation) then the substrate (50 µl) was added. The incubation was carried out for 30 min at 37°C (¹⁴C-5-HT, 5 µM) or for 10 min at 37°C (¹⁴C-PEA, 0.5 µM).

The reaction was stopped by adding 0.2 ml HCl or perchloric acid. After centrifugation, the deaminated metabolites were extracted with 3 ml of diethylether (5-HT) or toluene (PEA) and the radioactive organic phase measured by liquid scintillation spectrometry at 90% efficiency. Radioactivity in the eluate indicates the production of neutral and acidic metabolites formed as a result of MAO activity.

The enzymatic activity was expressed as nmoles of substrate transformed/mg protein/min. The activity of MAO in the sample was expressed as a percentage of control activity in the absence of inhibitors after subtraction of appropriate blank values.

The drug inhibition curves were obtained from at least eight different concentrations, each in duplicate (10⁻¹⁰ to 10⁻⁵ M) and the IC₅₀ values (the drug concentration inhibiting 50% enzyme activity) with confidence intervals determined using non linear regression analysis (best fitting aided-computer program).

### Example 3

### In vitro MAO-B inhibition reversibility studies

To investigate whether the test compound was an irreversible or a reversible MAO-B inhibitor, the inhibition of the enzymatic activity was assessed using two experimental protocols:
*- a) time-dependent experiments:*
   the time-dependent association kinetics were measured as the IC₅₀ values after 0 or 30 min enzyme-inhibitor incubation. For mechanism-based irreversible inhibitors that act by blocking the enzyme catalytic site, the inhibitory potency increases with incubation time. The absence of significant difference between IC₅₀ with or without preincubation is indicative for reversible inhibitors.
*- b) washing experiments:*
   rat brain mitochondria (enzyme) and test compound (inhibitor) were preincubated for 30 min at 37°C before being subjected to repeated centrifugations and resuspensions. MAO-B activity was assessed immediately and after each centrifugation resuspension cycles (1-5). Percentage inhibition was calculated with respect to samples treated in the same manner but in the absence of inhibitor.

The recovery of MAO-B activity after washings of the enzyme-test compound complex is indicative for reversible inhibitors.

### Example 4

### Ex vivo MAO-B inhibition

Test compounds were administered orally to male C57BL mice (Harlan, Italy, 25-27 g) at the single dose of 20 mg/Kg. At different time intervals (1, 2, 4, 8 and 24 h), animals were sacrificed, brains removed, cortices dissected out and stored at -80°C. Crude homogenates (0.5%) were prepared in 0.1 M phosphate buffer pH 7.4 and freshly used. MAO-A and MAO-B activity were assessed as above described.

### Example 5

### In vitro binding of ³H-batrachotoxin in rat brain membranes

*Membrane preparations (P2 fraction)* Male Wistar rats (Harlan, Italy - 175-200 g) were sacrificed under light anaesthesia and brains were rapidly removed and cortex was dissected out, homogenized in 10 vol. of ice-cold 0.25 M sucrose buffer (50 mM Tris·HCl, pH 7.4). The crude homogenate was centrifuged at 3250 rpm for 10 min and the supernatant recovered. The pellet was homogenized and centrifuged again and the two supernatants were pooled and centrifuged at 14750 rpm for 10 min, +4°C. The resulting pellet was stored at -20°C until use.

*Binding assay.* The pellet was resuspended in 50 mM Hepes buffer, pH 7.4 containing 0.8 mM MgSO₄, 5.4 mM KCl, 5.5 mM glucose and 130 mM choline using Polytron PT10. The binding assay was carried out in 0.25 ml final volume containing 50 µl of membrane preparation (ca 200 µg of protein), 50 µl of ³H-batrachotoxin ligand (10 nM), 50 µl of TTX (1 µM), 50 µl of scorpion toxin (37.5µg/ml) and 50 µl of test compound or buffer or 300 µM of veratridine to determine non specific binding. The binding assay was performed at 37°C for 30 min and stopped by rapid filtration under vacuum through Whatman GF/B glass fiber filters. Filters (pre-soaked with polyethylenimine 0.1%) were washed with 3x5 ml of ice-cold buffer and placed in picovials containing scintillation cocktail (Filter Count, Packard). Radioactivity bound was measured by liquid scintillation spectrometry at 45% efficiency.

*Data analysis.* The IC₅₀ was calculated from displacement curves by computer program LIGAND (McPherson, J. Pharmacol. Methods, 14, 213. 1985). The displacement curves were obtained using at least 9 concentrations, each in duplicate, covering a 100000 fold range.

### Example 6

### In vitro binding of ³H-nitrendipine in rat brain membranes

*Membrane preparations* Male Wistar rats (Harlan, Italy - 175-200 g) were sacrificed under light anaesthesia and brains were rapidly removed and cortex was dissected out, homogenized in 10 vol. of ice-cold 50 mM Tris•HCl, pH 7.7 using Polytron PT10. The crude homogenate was centrifuged at 50000xg for 10 min. The pellet was homogenized and centrifuged twice in fresh buffer at 50000xg for 10 min, +4°C. The resulting pellet was stored at - 20°C until use.

*Binding assay.* The pellet was resuspended in 50 mM Tris•HCl, pH 7.7 using Polytron PT10. The binding assay was carried out in 1 ml final volume containing 900 µl of membrane preparation (ca 700 µg of protein), 50 µl of ³H-nitrendipine (0.15 nM), and 50 µl of test compound or buffer or 1 µM of nifedipine to determine non specific binding. The binding assay was performed at 25°C for 45 min and stopped by rapid filtration under vacuum through Whatman GF/B glass fiber filters. Filters were washed with 3x5 ml of ice-cold buffer and placed in picovials containing scintillation cocktail (Filter Count, Packard). Radioactivity bound was measured by liquid scintillation spectrometry at 45% efficiency.

*Data analysis.* The IC₅₀ was calculated from displacement curves by computer program LIGAND (McPherson, J. Pharmacol. Methods, 14, 213. 1985). The displacement curves were obtained using at least 9 concentrations, each in duplicate, covering a 100000 fold range.

### Example 7

### Calcium influx assay

IMR32 human neuroblastoma cells constitutively possess both L and N type channels. Under differentiating conditions, IMR32 preferentially express on the membrane surface N-type calcium channels. The remaining L-type calcium channels were blocked using the selective L type blocker, nifedipine. In these experimental conditions, only N-type channels can be detected.

IMR32 cells were differentiated using 1 mM dibutyrril-cAMP and 2.5 *µ*M bromodeoxyuridine for 8 days (4 times) in 225 cm² flask, then detached, seeded at 200,000 cells/well on 96 polilysine-coated plates and further incubated for 18-24 h in the presence of differentiating buffer before use.

The Ca²⁺ Kit Assay (Molecular Devices), based on a fluorescent calcium indicator 485-535 nm wawelength, was used.

Differentiated cells were incubated with dye for 30 min at 37°C. Then, nifedipine alone (1 *µ*M) or in the presence of ω-conotoxin or test compounds were added for further 15 min.

The fluorescence (485-535nm) was measured before and after (30-40 sec) the automated injection of 100 mM KCl depolarizing solution using a Victor plate reader (Perkin Elmer).

### Example 8

### Maximal electroshock test in rats and mice

Wistar rats received an electroshock (160 mA for 0.2 s with a pulse train of 60 Hz having a pulse duration of 0.4 ms; ECT unit model 7801, Ugo Basile, Comerio, Italy) through intra-aural clip electrodes sufficient to produce a hindlimb tonic extensor response in at least 97% of control animals.

Mice received a 28 mA shock of 0.7 s with a pulse train of 80 Hz having a pulse duration of 0.4 ms. Several doses of the test compound and standard AEDs were administered to groups of 10 to 20 mice or rats per dose in a volume of 5 ml/kg 60 min p.o. or 30 min i.p. before induction of MES to calculate ED₅₀ values. The complete suppression of the hindlimb tonic extensor component of seizures was taken as evidence of anticonvulsant activity.

### Example 9

### Mice Formalin Test

According to a modified protocol from Rosland et al., (1990) mice were injected subcutaneously (s.c.) with 20 µl of 2.7% solution of formalin into the plantar surface of left hindpaw and placed immediately into clear PVC observation chambers (23 x 12 x 13 cm). Pain behaviour was quantified by counting the cumulative licking time (s) of the injected paw. Measurements were taken during the early phase (0-5 min) and late phase (30-40 min) after formalin injection (Tjolsen et al. 1992).

The test compound was administered p.o. 15 min before formalin injection in a volume of 10 ml/kg body weight to groups of 10 mice per dose. Control group was treated with vehicle.

### Example 10

### Carragenan model of inflammation

Male Wistar rats of 175-200 grams were used.

The left hind paw was injected with 100 µl of carrageenan (2% w/v in saline). Compounds of the invention (30 mg/kg), indomethacin (5 mg/kg) or control vehicle (such as distilled water) were orally administered 1 h before carrageenan injection. The paw volume was measured with a plethysmometer (Ugo Basile) immediately before (basal) and 1, 2, 3, 4 and 5 h after the carrageenan injection.

## Claims

1. A compound of general formula I 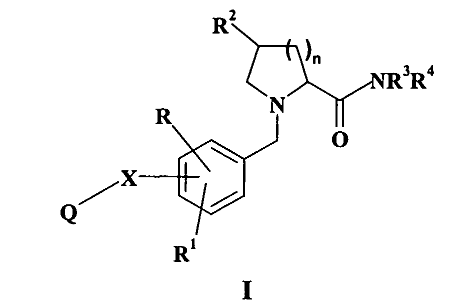 wherein
Q is C₃-C₈ alkyl, or C₁-C₈ alkyl substituted by phenoxy or saturated or unsaturated 5 to 7 membered ring optionally containing one or more heteroatoms chosen independently from nitrogen, oxygen or sulphur, the rings being optionally substituted by one or more halogen atoms, trifluoromethyl, C₁-C₆ alkyl, hydroxyl, C₁-C₆ alkoxy;
X is an oxygen, sulphur or a NR⁵ group;
R, R¹ are independently hydrogen, C₁-C₆ alkyl, halogen, trifluoromethyl, hydroxy or C₁-C₆ alkoxy;
R², is hydrogen or hydroxyl;
R³, R⁴, R⁵ are independently hydrogen or straight or branched C₁-C₃ alkyl;
n is an integer from 1 to 3
and pharmaceutically acceptable salts thereof.

2. A compound according to claim 1 selected from the group consisting of:
(S)-1-[2-(3-Fluoro-benzyloxy)-benzyl]-pyrrolidine-2-carboxylic acid amide;
(S)-1-[2-(3-Chloro-benzyloxy)-benzyl]-pyrrolidine-2-carboxylic acid amide;
(S)-1-[3-(3-Fluoro-benzyloxy)-benzyl]-pyrrolidine-2-carboxylic acid amide;
(S)-1-[3-(3-Chloro-benzyloxy)-benzyl]-pyrrolidine-2-carboxylic acid amide;
(S)-1-[4-(3-Fluoro-benzyloxy)-benzyl]-pyrrolidine-2-carboxylic acid amide;
(S)-1-[4-(3-Chloro-benzyloxy)-benzyl]-pyrrolidine-2-carboxylic acid amide;
(2S)-1-[4-(3-Fluoro-benzyloxy)-benzyl]-4-hydroxy-pyrrolidine-2-carboxylic acid amide;
(2S)-1-[4-(3-Chloro-benzyloxy)-benzyl]-4-hydroxy-pyrrolidine-2-carboxylic acid amide;
(S)-1-[4-(3-Fluoro-benzyloxy)-benzyl]-piperidine-2-carboxylic acid amide;
(S)-1-[4-(3-Chloro-benzyloxy)-benzyl]-piperidine-2-carboxylic acid amide;
(S)-1-[4-(3-Fluoro-benzyloxy)-benzyl]-azepine-2-carboxylic acid amide;
(S)-1-[4-(3-Chloro-benzyloxy)-benzyl]-azepine-2-carboxylic acid amide;
(S)-1-[4-(3-Chloro-benzyloxy)-benzyl]-pyrrolidine-2-carboxylic acid N-methyl amide;
(S)-1-[4-(3-Trifluoromethyl-benzyloxy)-benzyl]-pyrrolidine-2-carboxylic acid amide;
(S)-1-[4-(3-Trichloromethyl-benzyloxy)-benzyl]-pyrrolidine-2-carboxylic acid amide;
(S)-1-[4-(3,4-di-Chloro-benzyloxy)-benzyl]-pyrrolidine-2-carboxylic acid amide;
(S)-1-[4-(3-Chloro-benzyloxy)-2-chlorobenzyl]-pyrrolidine-2-carboxylic acid amide;
(S)-1-[4-(2-Fluoro-benzyloxy)-3-chlorobenzyl]-pyrrolidine-2-carboxylic acid amide;
(S)-1-[4-(2-Fluoro-benzyloxy)-2-methoxybenzyl]-pyrrolidine-2-carboxylic acid amide;
(S)-1-[4-(2-Fluoro-benzyloxy)-3-methoxybenzyl]-pyrrolidine-2-carboxylic acid amide;
(S)-1-[4-(2-Fluoro-benzyloxy)-3-bromo-5-methoxybenzyl]-pyrrolidine-2-carboxylic acid amide;
(S)-1-[4-(2-Fluoro-benzyloxy)-3-fluorobenzyl]-pyrrolidine-2-carboxylic acid amide;
(S)-1-[4-(3-Trifluoromethyl-benzyloxy)-2-chlorobenzyl]-pyrrolidine-2-carboxylic acid amide;
(S)-1-[4-(Pyridin-2-ylmethoxy)-benzyl]-pyrrolidine-2-carboxylic acid amide;
(S)-1-[4-(Pyridin-3-ylmethoxy)-benzyl]-pyrrolidine-2-carboxylic acid amide;
(S)-1-[4-(Pyridin-4-ylmethoxy)-benzyl]-pyrrolidine-2-carboxylic acid amide;
and the corresponding (R) enantiomers or pharmaceutically acceptable salts thereof.

3. A process for the preparation of a compound of formula **I**, as defined in claim 1, or a pharmaceutically acceptable salt thereof, the process comprising:
a) reaction of a compound of formula **II** 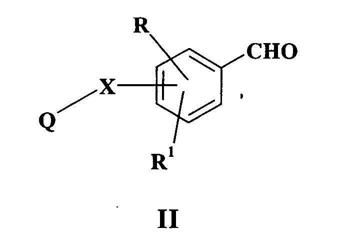 wherein Q, X, R and R¹ are as defined in claim 1
with a compound of formula **III,** in the presence of a reducing agent 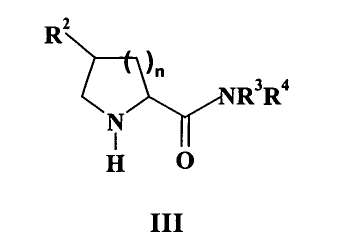 wherein R², R³, R⁴ and n are as defined in claim 1, or
b) reaction of a compound of formula **IV,** 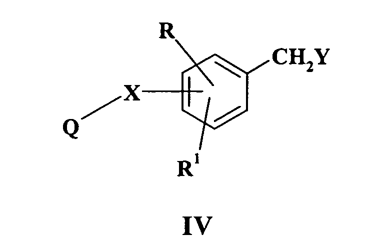
wherein Q, X, R and R¹ are as defined in claim 1 and Y is a halogen atom or a O-EWG group, where the EWG means an electron withdrawing group, with compounds of formula **III** and, if desired, converting a compound of the invention into another compound of the invention and/or, if desired, converting a compound of the invention into a pharmaceutically acceptable salt and/or, if desired, converting a salt into a free compound.

4. A pharmaceutical composition containing, as an active principle, a compound of formula **I**, as defined in claim 1, or a pharmaceutically acceptable salt thereof in addition to a suitable carrier and/or diluent and optionally to other therapeutic agents.

5. A compound of formula **I,** as defined in claim 1, or a pharmaceutically acceptable salt thereof, for use as an active therapeutic substance.
